# EUROPEAN PATENT SPECIFICATION

(11) **EP 1 445 611 B1**
(45) Date of publication and mention of the grant of the patent: **07.03.2012**
(21) Application number: 04100285.8
(22) Date of filing: 28.01.2004
(51) Int. Cl.: G01N 33/18

(54) **Apparatus and method for measuring the quality of water**
Vorrichtung und Verfahren zum Messen der Wasserqualität
Appareil et procédé pour mesurer la qualité de l'eau

(30) Priority: 04.02.2003 FI 20030169
(43) Date of publication of application: 11.08.2004
(73) Proprietor: Luode Consulting Oy, 00180 Helsinki (FI)
(72) Inventor: Lindfors, Antti, 00180, Helsinki (FI)
(74) Representative: Valkeiskangas, Tapio Lassi Paavali

(56) References cited:
- US-A- 5 831 727
- US-B1- 6 444 119
- PATENT ABSTRACTS OF JAPAN vol. 2000, no. 13, 5 February 2001 (2001-02-05) & JP 2000 275241 A (HITACHI LTD), 6 October 2000 (2000-10-06)
- PATENT ABSTRACTS OF JAPAN vol. 0170, no. 23 (P-1470), 18 January 1993 (1993-01-18) & JP 4 248464 A (TOSHIBA CORP), 3 September 1992 (1992-09-03)

## Description

### BACKGROUND OF THE INVENTION

The invention relates to an apparatus according to the preamble of claim 1 for the continuous measurement of the quality of water and to a method according to the preamble of claim 11 for the continuous measurement of the quality of water.

According to prior art, point measurements are used for measuring the quality of water in waterways and water areas, whereby a separate water sample is taken from each selected measuring point, and the sample is analyzed. When samples are collected, the vessel employed always has to be moved to a new measuring point and stopped for the time of the collection of the measurement sample, after which the travel continues again to a new measuring point. The thus obtained measuring results are composed of measuring results obtained at individual sites, and they do not give a fully realistic idea of the localized values of the variables to be measured and the changes in the values between the different measuring points.

DD146855 discloses an apparatus which can be attached to a boat for continuous monitoring of water quality, comprising a suction head and hose assembly whereby the depth at which the suction head hangs may be altered. The hose passes between two rollers which forms part of a measuring device capable of measuring the amount of hose run out to the nearest centimetre.

A problem in the above-described arrangement is that from 20 to 40 individual point measurements can be performed within 24 hours. This is not sufficient, since for instance when the quality of water in large water areas is measured, a large number of measurements has to be made in order to obtain a map illustrating the quality of water in the entire water area from the localized values of the quality of water, the map also showing the change in the quality of water between the measuring points. In addition, making point measurements takes up a lot of time, since a new measuring point always has to be reached and the vessel stopped. This may distort the results, since the quality of water may vary in time during lengthy measurements. Making point measurements is also expensive due to the long time taken up thereby. Associating individual samples automatically with location information is technically difficult, wherefore the measuring results have to be subjected to significant further processing when point measurements are used.

Continuous measurement of the quality of water in a waterway, water area and the like was not possible previously because of air bubbles entrapped in the measurement line in connection with water extraction. Air bubbles distort particularly optical measurements and conductivity measurements, which are used to determine the mixing of water masses and the concentrations of the substances contained therein. Air/gas bubbles may also cause problems in the measurement of the liquids of industrial processes, i.e. the present invention is utilized therein for removing bubbles. For the sake of simplicity, only water will be referred to hereinafter, whereby it also refers to other liquids.

### BRIEF DESCRIPTION OF THE INVENTION

The object of the invention is thus to provide an apparatus according to the preamble of claim 1 and a method according to the preamble of claim 11 for continuous measurement of the quality of water or a liquid associated with an industrial process to solve the above problems. The object of the invention is achieved with an apparatus, which is characterized in that the apparatus comprises a bubble removal chamber installed in a measurement line before measuring sensors for removing air bubbles contained by the water led into the measurement line.

The object of the invention is further achieved by a method, which is characterized by comprising the following steps:
drawing water to be measured into the apparatus via a water intake pipe fitting,
leading the water drawn into the apparatus along a measurement line into a bubble removal chamber for removing air and/or gas bubbles possibly existing in the water,
leading the water, cleared of air and/or gas bubbles, along the measurement line further to at least one measuring sensor for making measurements of the water led into the apparatus,
displaying and/or storing and/or processing data measured with the measuring sensors by means of data management means, and
leading the water along the measurement line via a water removal pipe fitting out of the apparatus.

Preferred embodiments of the invention are described in the dependent claims.

The invention is based on allowing the water to be measured to flow continuously through the measurement apparatus.The apparatus is installed in a seagoing vessel by placing the tubular water intake pipe fitting of the apparatus in water and by directing its orifice end in the travel direction, whereby the water to be analyzed flows continuously into the apparatus when the vessel is driven. The pressure of the water led into the apparatus is equalized with a depressurization pump. Air bubbles hindering and even preventing reliable measuring results from being obtained exist or are generated in the water that is taken in. For this reason, the water is led to a bubble removal chamber before the measuring sensors, wherein the water is forced into a rotating movement, whereby the bubbles therein are driven to the middle of the whirlpool created, from which they are removed. The water, cleared of bubbles, is in turn led forward to measuring sensors that make the necessary measurements and transmit the results obtained forward to data management means that display and/or store and/or process the measuring results obtained. The water discharged from the measuring sensors is led further out of the apparatus. The combination of location data means with the apparatus enables automatic and real-time coupling of the measuring results obtained with the location data obtained.

An advantage of the method and arrangement of the invention is that it enables continuous reliable measurement of the quality of water in a waterway, water area and the like for instance when a vessel is driven. In this case, 5,000 to 15,000 reliable individual measuring results are obtained in 2 to 5 hours, depending on the driving speed of the vessel or a corresponding collecting speed. In this case, the measurement collection speed can also be made high, thereby avoiding a distortion of results in time caused by a long measuring time and an increase in cost. In addition, this minimizes the further processing of the material, since the location data on the measurements can also be automatically associated with the measuring results obtained.

In a preferred embodiment of the apparatus of the invention, the apparatus also comprises a depressurization pump for equalizing the water pressure in the measurement line to the desired level.

In another preferred embodiment of the invention, the apparatus further comprises location data means for associating the measuring results obtained from the water to be measured with the location data.

Furthermore, in a preferred embodiment of the apparatus of the invention, the bubble removal chamber comprises an inlet pipe fitting for applying water into the bubble removal chamber, a removal pipe fitting for discharging the water from the bubble removal chamber, and a bubble removal pipe fitting for removing bubbles from the bubble removal chamber. In this case, the bubble removal chamber is designed and the inlet pipe fitting of the bubble removal chamber is placed such that the water flowing into the chamber is forced into a rotating movement in the chamber.

In a second preferred embodiment of the apparatus of the invention, the cross-section of the bubble removal chamber in the flow direction of the water flowing into the bubble removal chamber via the inlet pipe fitting is substantially circular, preferably cylindrical or conical.

Furthermore, in another preferred embodiment of the apparatus of the present invention, the inlet pipe fitting of the bubble removal chamber is placed in the lower part of the jacket partition of the bubble removal chamber above the bottom partition tangentially to the jacket partition such that the water entering the chamber is forced into a rotating movement, the bubble removal pipe fitting being placed in the bubble removal chamber coaxially with the axis of the whirlpool created in the bubble removal chamber and the removal pipe fitting being placed in the bottom partition of the bubble removal chamber.

In a preferred embodiment of the method of the invention, water pressure is controlled with a depressurization pump before leading it to a bubble removal chamber for equalizing the water pressure to the desired level.

In another embodiment of the method of the present invention, location data means are connected to at least one measuring sensor and/or the location management means for associating the measuring results with the location data.

Furthermore, in a preferred embodiment of the method of the invention, water is led to the bubble removal chamber such that the water in the chamber is forced into a rotating movement, whereby any air and/or gas bubbles therein are driven to the middle of the whirlpool created, from which they are removed.

Furthermore, in another preferred embodiment of the method of the invention, the water to be measured is drawn into the apparatus by moving at least the water intake pipe fitting relative to the water in the waterway, water area or the like and/or by using a pump to suck water from the waterway, water area or the like.

### BRIEF DESCRIPTION OF THE FIGURES

In the following, preferred embodiments of the invention will be described in detail with reference to the accompanying drawings, in which
Figure 1 shows a measurement apparatus according to an embodiment of the present invention.
Figure 1a is a side view of a bubble removal chamber according to an embodiment of the measurement apparatus of the present invention.
Figure 2b is a top view of a bubble removal chamber according to an embodiment of the measurement apparatus of the present invention.
Figure 3 shows a measurement apparatus according to an embodiment of the present invention installed in a vessel.

### DETAILED DESCRIPTION OF THE INVENTION

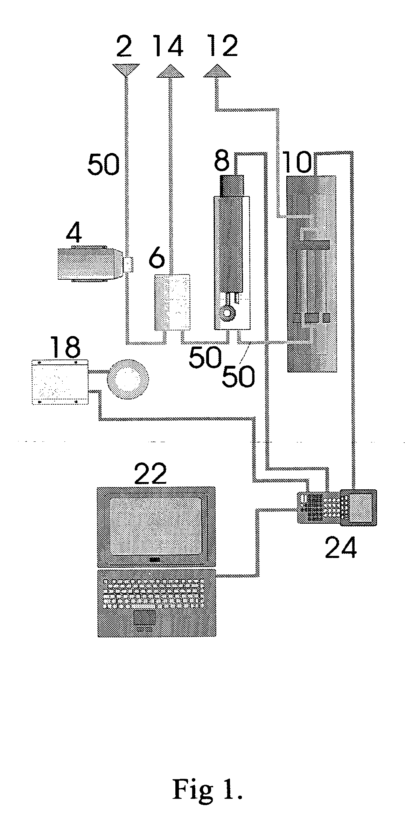

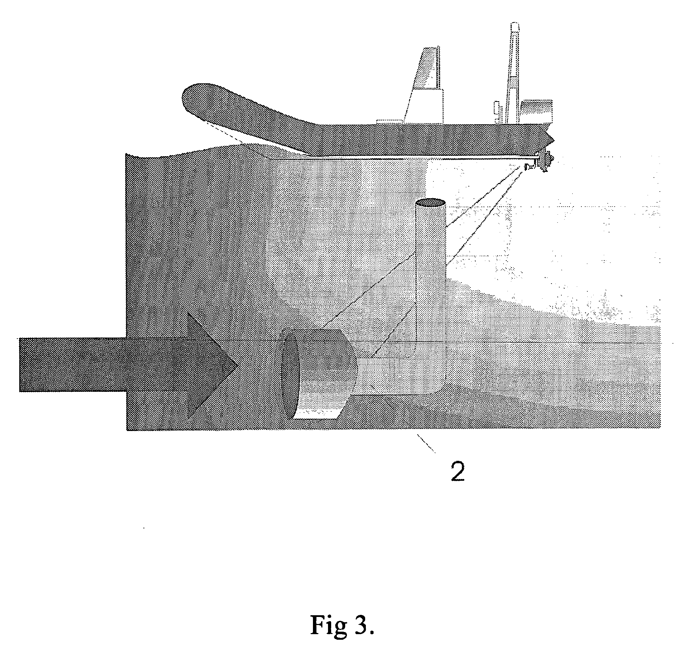

Figure 1 shows a diagram of a measurement apparatus according to an embodiment of the present invention installed in a vessel travelling in a waterway, water area or the like. The apparatus comprises a tubular water inlet pipe fitting 2, via which water to be analyzed is led to a measurement line 50. The orifice of the inlet pipe fitting 2 can be widened with a funnel, muffle or the like to achieve a sufficient water inflow. The diameter of the widening is typically twice as large as the diameter of the tube of the measurement line. During measurement, the inlet pipe fitting 2 is placed in water such that its open orifice is directed substantially in the travel direction of the vessel, whereby water is allowed to flow via the orifice of the inlet pipe fitting 2 into the measurement line 50 when the vessel is moving. In this case, the end of the inlet pipe fitting 2 is typically bent forward in accordance with Figure 3.

The inlet pipe fitting 2 and the measurement line 50 can be fastened fixedly or detachably to the vessel. The inlet pipe fitting 2 can be placed for instance at the rear of the vessel or, alternatively, at a flank of the vessel. The parts located at the inlet pipe fitting 2 below the water level are designed such that the travel path of the water is free from sharp edges or forms that could prevent the water from flowing smoothly into the apparatus. Optionally, the inlet pipe fitting 3 can be made either fixed such that the depth of the inlet pipe fitting 2 is constant or it can be made adjustable enabling the adjustment of the measuring depth as necessary.

In association with the above-described water intake, bubbles are created in the water that is drawn in, and they prevent reliable measurements from being made, whereby optical measurements and conductivity measurements for determining the mixing of the water masses and the concentrations of the substances therein are impossible. Air bubbles gaining access to the measurement line are the reason preventing continuous measurements with conventional apparatuses, since air bubbles are not generated during single point measurements. For this reason, said air bubbles have to be removed from the water flowing in the measurement line 50 before the water is led to the measuring sensors 8, 10.

The water to be analyzed in the inlet pipe fitting 2 is led to a depressurization pump 4, which generates a constant pressure in the measurement line, typically from 2 to 8 bar. In addition, the depressurization pump 4 enables changes in the vessel's driving speed and stopping the vessel without jeopardizing a sufficiently even flow of the water entering the apparatus. The pump 4 also removes the variation caused by external circumstances, e.g. weather, and generates a sufficient water pressure in all situations for achieving efficient bubble removal.

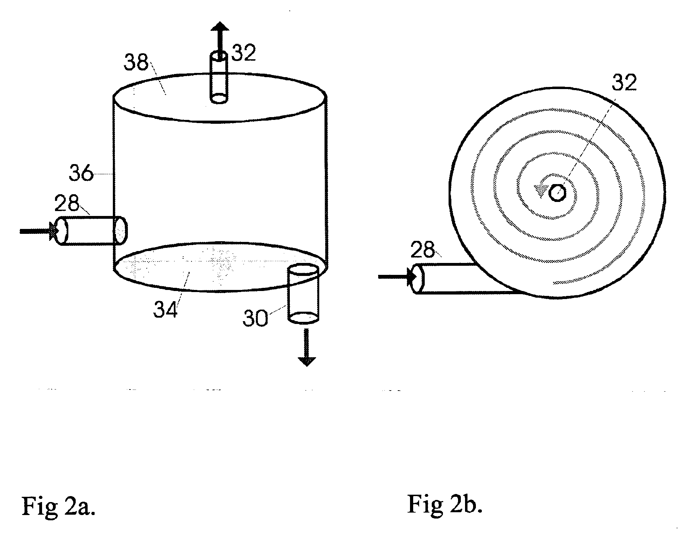

In accordance with Figure 1, the water is led from the depressurization pump along the measurement line 50 to a bubble removal chamber 6. The water, at the high pressure generated by the depressurization pump 4, is led into the bubble removal chamber 6 along an inlet pipe fitting 28 such that it enters the chamber 6 slightly above its bottom partition 34, preferably a few centimetres above the bottom partition in accordance with Figure 2a. The arrows in Figure 2 show the travel directions of the water. The bubble removal chamber 6 is designed such that the water entering it at pressure is forced into an intense rotating movement in accordance with Figure 2b. In this case, the inlet pipe fitting 28 of the bubble chamber 6 also has to be placed in a manner enhancing the generation of a whirlpool in the chamber 6.

Figures 2a and 2b show a preferred embodiment of the bubble removal chamber 6. In these figures, the bubble removal chamber 6 is substantially cylindrical, the circular cross-section of the jacket partition 36 of the chamber 6 enabling the subjecting of the water entering the chamber to a rotating movement. The shape of the bubble removal chamber 6 may also be conical, the cross-section of its jacket partition 36 diminishing towards the lower end or upper end of the chamber. The cross-section of the jacket partition 36 of the chamber 6 does not necessarily have to be circular; instead, it may be for instance a polygon or symmetrical or an eccentric ellipse or the like, as long as it enables the efficient subjecting of the water entering the bubble removal chamber 6 to a rotating movement.

Besides the shape of the bubble removal chamber 6, another important factor affecting the rotation of the water is the placement and orientation of the inlet pipe fitting 28 of the chamber 6. In this exemplary embodiment, the inlet pipe fitting 28 is placed and oriented substantially tangentially in respect of the wall the jacket partition 36 of the chamber 6 slightly above the bottom partition 38 of the chamber 6 in accordance with Figure 2a. The thus implemented lead-through of the inlet pipe fitting 28 through the wall of the bubble removal chamber 6 together with an advantageously designed chamber 6 and the water entering the chamber 6 at high pressure generate an intense rotating movement of the water inside the bubble removal chamber 6.

The inlet pipe fitting 28 can be placed and oriented in the chamber also in some alternative manner. For example, in a situation where the cross-section of the jacket partition 36 of the chamber 6 is polygonal, the inlet pipe fitting 28 can be placed in one corner of the polygon in the direction of the cross-section of the jacket partition 36 such that it is parallel with the side of the following polygon in the flow direction of the water. The tangential placement and orientation of the inlet pipe fitting 28 can be utilized in all chambers 6 of the jacket portion 36 having arched cross-sections. The inlet pipe fitting 28 may also be oriented either sloping upwards or downwards towards the cover partition 38 or the bottom partition 34, respectively, of the bubble removal chamber 6.

In accordance with the above-described solutions, the water to be led inside the bubble removal chamber 6 is forced into an intense rotating movement in accordance with Figure 2b. Due to the rotating movement generated, a strong and rapid whirlpool is generated in the bubble removal chamber 6, whereby the air/gas bubbles in the water drawn into the measurement line 50 are driven to the middle of the whirlpool as a result of the rotating movement of the water. The bubbles absorbed into the middle of the whirlpool are removed from the bubble removal chamber 6 via the bubble removal pipe fitting 32 led through its cover partition 38. The bubble removal pipe fitting 32 is placed at the middle axis of the jacket partition 36 of the bubble removal chamber 6, the partition having a circular cross-section, at which axis the middle of the whirlpool is also placed. The bubbles driven into the middle of the whirlpool are now removed from the chamber 6 along the bubble removal pipe fitting 32. Some water is also removed from the apparatus along with the bubbles, since the removal of only bubbles is impossible. The point in the placement of the bubble removal pipe fitting 32 is that it is exactly in line, coaxially, with the middle axis of the whirlpool created in the bubble removal chamber 6, i.e. with the whirlpool axis in the manner shown in Figure 2b. The shape of the chamber 6 can be used to efficiently affect the shape and strength of the whirlpool created.

Depending on the above-described shape of the bubble removal chamber 6, a special location of the bubble removal pipe fitting 32 has to be defined for each chamber type such that it is, irrespective of chamber type or shape, substantially aligned with, preferably co-axially with the middle of the whirlpool created. This ensures efficient bubble removal from the bubble removal chamber 6. The bubble removal pipe fitting 32 is preferably placed in the cover partition of the chamber 6, since air bubbles inherently tend to rise upwards in water, but in some cases, the bubble removal pipe fitting 32 can be placed also in the bottom partition 34 of the chamber 6. If need be, bubble removal can be enhanced by placing a pump in the bubble removal pipe fitting 32, the pump sucking water and bubbles driven into the middle of the whirlpool out of the chamber 6.

The water driven to the edges of the bubble removal chamber 6 is substantially bubble-free and accordingly suitable for continuous water quality measurement. Since bubbles also tend to rise upwards in water, in this exemplary embodiment, the removal pipe fitting 30 is placed in the bottom partition 34 of the bubble removal chamber 6 near the edge that joins the bottom partition 34 and the jacket partition 36 together, as is shown in Figure 2a. The removal pipe fitting 30 may also be placed for instance at a corresponding point of the cover partition 38 or in the jacket partition, as long as air bubbles are prevented from entering the measurement line 50 from the chamber 6. A pump for enhancing the removal of water can also be used in association with the removal pipe fitting 30.

The volume of the bubble removal chamber 6 is made as small as possible in order for the changes in time and in place of the water mass to be measured not to disappear as a result of mixing. For its part, the chamber 6 also equalizes the pressure changes in the water ending up in the measurement line 50, the changes being caused by waves in the waterway or the like and other external circumstances. The bubble removal chamber 6 also serves as an auxiliary system should the entire water intake pipe fitting 2 momentarily rise above the water level for instance in a strong wind or as the vessel heels over.

From the bubble removal chamber 6, the bubble-free water is led along the measurement line 50 into the measuring sensors 8, 10. The sensors 8, 10 are placed in succession in the measurement line 50 as close to each another as possible in order for the water to be analyzed to flow as quickly as possible and simultaneously through all sensors. The measuring sensors 8, 10 may be both process-monitoring sensors intended for industrial use and operated by the flow-through method and separate optical devices, which are intended for the detection of impurities in the water and in which the sample is conveyed through glass or plastic measuring cuvettes. The cuvettes are designed in a manner enabling a continuous flow of the water to be analyzed through them. The composition of the parameters to be measured can be changed as necessary by adding or removing sensors in use.

The measuring parameters obtained from the measuring sensors 8, 10 are stored in a data logger from which they are transferred to a computer for processing or storage or onto a screen for direct display. The collected measuring results obtained are displayed, stored and processed with the data management means 22, 24, or they can be printed out directly on paper. The measuring results can also be transmitted directly into a data network, from where they are further conveyed via a network server to the receiver.

The location data means 18, 20 for associating the measuring results with the location data are also preferably connected to the measuring sensors 8, 10 and the data management means 22, 24. For example, the GPS or DGPS systems or another corresponding system may serve as the location data means. Location data can also be obtained from the location data system of the vessel. The location data are used to carry out migration studies, dilution studies and to create different water quality maps or utilize the obtained measuring results associated with the location data in the design of various facilities to be built by water areas.

Eventually, the water discharged from the measuring sensors is led out of the apparatus along the measurement line 50 and the water removal pipe fitting 12.

When the above-described apparatus is used, the water to be measured is drawn into the apparatus via the water inlet pipe fitting 2, after which the water drawn into the measurement line 50 is led along the measurement line 50 into the bubble removal chamber 6 for removal of any air and/or gas bubbles in the water such that the water is forced into a rotating movement in the chamber, whereby the bubbles in the water are driven to the middle of the whirlpool created, from which they are removed. Next, the water, cleared of gas bubbles, is led along the measurement line 50 further to at least one measuring sensor 8, 10 for making measurements of the water led into the apparatus. The measuring results are displayed and/or stored and/or processed by means of data management means, and associated with the location data obtained by means of the location data means 18, 20, and finally the water is led along the measurement line 50 via the water outlet pipe fitting 12 out of the apparatus.

In the above-described manner, continuous water quality measurement can be made as the vessel travels in a water area. The above-described apparatus allows the vessel to move at the desired speed, e.g. 0 to 50 km/h, and yet reliable measuring results of the quality of water are obtained simultaneously. In addition, a large number of measuring results are obtained in a short period of time, as many as 5,000 to 15,000 in 2 to 5 hours. Accordingly, the advantages of the present apparatus and method are extremely significant compared with collecting samples by the conventional point measurement.

The above-described apparatus and method are also applicable to bubble removal in the measurement of industrial process liquids. In this case, the liquid to be analyzed is taken from some process step into the measurement apparatus.

It is obvious to a person skilled in the art that as technology advances, the basic idea of the invention can be implemented in a variety of ways. The invention and its embodiments are thus not limited to the above examples, but may vary within the claims.

## Claims

1. An apparatus for continuous measurement of the quality of water in a waterway, water area or the like from a moving seagoing vessel, in that the apparatus comprises a water inlet pipe fitting (2) installed from the vessel in a waterway, water area or the like for leading the water to be measured to the apparatus by means of a measurement line (50) as the vessel is moving, at least one measuring sensor (8, 10) for making measurements of the water led into the apparatus, data management means (22, 24) for displaying and/or storing and/or processing the measured data, and a water outlet pipe fitting (14) for discharging the water passed through the measurement line (50) out of the apparatus, and a bubble removal chamber (6) installed in the measurement line (50) before the measuring sensors (8, 10) for removal of air and/or gas bubbles contained by the water led into the measurement line (50) for continuous measurement of the quality of water in the waterway, water area or the like.

2. An apparatus as claimed in claim 1, **characterized in that** it further comprises a depressurization pump (4) for equalizing the water pressure conveyed in the measurement line (50).

3. An apparatus as claimed in claim 1 or 2, **characterized in that** it further comprises location data means (18, 20) for associating the measuring results obtained from the water to be measured with location data.

4. An apparatus as claimed in any one of claims 1 to 3, **characterized in that** a bubble removal chamber (6) comprises an inlet pipe fitting (28) for feeding water into the bubble removal chamber (6), an outlet pipe fitting (30) for discharging water from the bubble removal chamber (6), and a bubble removal pipe fitting (32) for removing bubbles from the bubble removal chamber (6).

5. An apparatus as claimed in any one of claims 1 to 4, **characterized in that** the bubble removal chamber (6) is designed and the inlet pipe fitting (28) of the bubble removal chamber (6) is placed such that the water flowing into the chamber (6) is forced into a rotating movement in the chamber (6).

6. An apparatus as claimed in any one of claims 1 to 5, **characterized in that** the cross-section of the bubble removal chamber (6) in the flow direction of the water entering the bubble removal chamber (6) via the inlet pipe fitting (28) is substantially circular.

7. An apparatus as claimed in claim 6, **characterized in that** the bubble removal chamber (6) is substantially cylindrical.

8. An apparatus as claimed in claim 6, **characterized in that** the bubble removal chamber (6) is substantially conical.

9. An apparatus as claimed in any one of claims 1 to 8, **characterized in that** the inlet pipe fitting (28) of the bubble removal chamber (6) is placed in the lower part of a jacket partition (36) of the bubble removal chamber (6) above a bottom partition (34) tangentially to the jacket partition (36) such that the water entering the chamber is forced into a rotating movement.

10. An apparatus as claimed in any one of claims 1 to 9, **characterized in that** the bubble removal pipe fitting (32) is placed in the bubble removal chamber (6) coaxially with the axis of the middle of a whirlpool created in the bubble removal chamber (6).

11. An apparatus as claimed in any one of claims 1 to 10, **characterized in that** the outlet pipe fitting (30) is placed in the bottom partition (30) of the bubble removal chamber (6).

12. A method for continuous measurement of the quality of water in a waterway, water area or the like from a moving seagoing vessel, comprising the following steps:
continuously drawing water to be measured from the waterway, water area or the like into the apparatus via a water intake pipe fitting (2) as the vessel is moving,
leading the water drawn into the apparatus along a measurement line (50) into a bubble removal chamber (6) for removing any air and/or gas bubbles in the water before making measurements of the water drawn into the apparatus,
leading the water, cleared of air and/or gas bubbles, along the measurement line (50) further to at least one measuring sensor (8, 10) for making continuous measurements of the water led into the apparatus,
displaying and/or storing and/or processing data measured with the measuring sensors (8, 10) by means of data management means (22, 24), and
leading the water along the measurement line (50) via a water removal pipe fitting (12) out of the apparatus.

13. A method as claimed in claim 12, **characterized by** controlling the water pressure with a depressurization pump (4) before leading it into the bubble removal chamber (6) for equalizing the water pressure to a desired level.

14. A method as claimed in claim 12 or 13, **characterized by** connecting location data means (18, 20) to at least one measuring sensor (8, 10) and/or the data management means (22, 24) for associating the measuring results with location data.

15. A method as claimed in any one of claims 12 to 14, **characterized by** leading the water to the bubble removal chamber (6) in a manner forcing the water into a rotating movement in the chamber (6), whereby any air and/or gas bubbles are driven into the middle of a whirlpool created, from which they are removed.

16. A method as claimed in any one of claims 12 to 15, **characterized by** drawing the water to be measured into the apparatus by moving at least the water inlet pipe fitting (2) relative to the water in the waterway, water area or the like and/or by using a pump to suck water from the waterway, water area or the like.

## Patentansprüche

1. Eine Vorrichtung zum kontinuierlichen Messen der Wasserqualität in einer Wasserstraße, einem Wassergebiet oder Ähnlichem von einem sich in Bewegung befindenden hochseetüchtigen Schiff aus **dadurch gekennzeichnet, dass** die Vorrichtung zur Wasserzuleitung einen Zuflussleitungsstutzen (2), der aus dem Schiff in die Wasserstraße, das Wassergebiet oder in Ähnliches eingeführt wird, zum Leiten des zu messenden Wassers in die Vorrichtung mit Hilfe einer Messungslinie (50) während das Schiff sich in Bewegung befindet, mindestens einen Messsensor (8, 10) zur Durchführung von Messungen des in die Vorrichtung geleiteten Wassers, Mittel (22, 24) zur Handhabung von Daten zum Zeigen und/oder Speichern und/oder Verarbeiten der gemessenen Daten, und einen für die Wasserableitung vorgesehenen Abflussleitungsstutzen (14) zum Ableiten des durch die Messungslinie (50) geleiteten Wassers aus der Vorrichtung heraus, und einen in die Messungslinie (50), vor den Messsensoren (8, 10) angeordneten Behälter (6) zur Entfernung von Luft- und/oder Gasbläschen, die in dem in die Messungslinie (50) geleiteten Wasser enthalten sind, zum kontinuierlichen Messen der Wasserqualität in einer Wasserstraße, einem Wassergebiet oder Ähnlichem, aufweist.

2. Eine Vorrichtung nach Patentanspruch 1 **dadurch gekennzeichnet, dass** sie weiter eine den Druck herabsetzende Pumpe (4) zur Ausgleichung des Druckes des in der Messungslinie (50) beförderten Wassers aufweist.

3. Eine Vorrichtung nach Patentanspruch 1 oder 2 **dadurch gekennzeichnet, dass** sie weiter Lagedatenmittel (18, 20), um die erhaltenen Messresultate von dem zu messenden Wasser mit Lagedaten in Verbindung zu bringen, aufweist.

4. Eine Vorrichtung nach einem beliebigen Patentanspruch 1 bis 3 **dadurch gekennzeichnet, dass** ein Behälter (6) zur Entfernung von Bläschen einen für die Wasserzuleitung vorgesehenen Zuflussleitungsstutzen (28) zur Zuleitung des Wassers in den Behälter (6) zur Entfernung von Bläschen, einen für die Wasserableitung vorgesehenen Abflussleitungsstutzen (30) zur Ableitung des Wassers aus dem Behälter (6) zur Entfernung von Bläschen, und einen für die Entfernung von Bläschen vorgesehenen Leitungsstutzen (32) zur Entfernung von Bläschen aus dem Behälter (6) zur Entfernung von Bläschen aufweist.

5. Eine Vorrichtung nach einem beliebigen Patentanspruch 1 bis 4 **dadurch gekennzeichnet, dass** der Behälter (6) zur Entfernung von Bläschen derart konstruiert und der Zuflussleitungsstutzen (28) des Behälters (6) zur Entfernung von Bläschen derart platziert ist, dass das in den Behälter (6) fließende Wasser in dem Behälter (6) in eine rotierende Bewegung gezwungen wird.

6. Eine Vorrichtung nach einem der Patentansprüche 1 bis 5 **dadurch gekennzeichnet, dass** der Querschnitt des Behälters (6) zur Entfernung von Bläschen in der Fließrichtung des in den Behälter (6) zur Entfernung von Bläschen durch den Zuflussleitungsstutzen (28) hineingeleiteten Wassers im Wesentlichen kreisförmig ist.

7. Eine Vorrichtung nach Patentanspruch 6 **dadurch gekennzeichnet, dass** der Behälter (6) zur Entfernung von Bläschen im Wesentlichen zylindrisch ist.

8. Eine Vorrichtung nach Patentanspruch 6 **dadurch gekennzeichnet, dass** der Behälter (6) zur Entfernung von Bläschen im Wesentlichen konisch ist.

9. Eine Vorrichtung nach einem der Patentansprüche 1 bis 8 **dadurch gekennzeichnet, dass** der Zuflussleitungsstutzen (28) des Behälters (6) zur Entfernung von Bläschen in den unteren Teil einer Ummantelung (36) des Behälters (6) zur Entfernung von Bläschen, oberhalb eines Bodenteils (34), tangential zur Ummantelung (36) platziert ist, so dass das Wasser in eine rotierende Bewegung gezwungen wird.

10. Eine Vorrichtung nach einem der Patentansprüche 1 bis 9 **dadurch gekennzeichnet, dass** der Leitungsstutzen (32) zur Entfernung von Bläschen in dem Behälter (6) zur Entfernung von Bläschen koaxial zur Achse der Mitte eines in dem Behälter (6) zur Entfernung von Bläschen entstandenen Strudels platziert ist.

11. Eine Vorrichtung nach einem der Patentansprüche 1 bis 10 **dadurch gekennzeichnet, dass** der Abflussleitungsstutzen (30) in dem Bodenteil (30) des Behälters (6) zur Entfernung von Bläschen platziert ist.

12. Ein Verfahren zum kontinuierlichen Messen der Wasserqualität in einer Wasserstraße, einem Wassergebiet oder Ähnlichem von einem sich in Bewegung befindenden hochseetüchtigen Schiff aus, wobei es folgende Schritte aufweist:
kontinuierlich zu messendes Wasser aus der Wasserstraße, dem Wassergebiet oder Ähnlichem durch einen Zuflussleitungsstutzen (2) zuführen, während das Schiff sich bewegt,
das in die Vorrichtung zugeleitete Wasser entlang der Messungslinie (50) in einen Behälter (6) zur Entfernung von Luft- und/oder Gasbläschen weiterleiten, bevor Messungen des in die Vorrichtung geleiteten Wassers durchgeführt werden,
das von Luft- und/oder Gasbläschen befreite Wasser, entlang der Messungslinie (50) zu mindestens einem Messsensor (8, 10) weiterleiten, um kontinuierliche Messungen des in die Vorrichtung geleiteten Wassers durchzuführen,
die mit den Messsensoren (8, 10) gemessenen Daten mit Hilfe von Mitteln (22, 24) zur Handhabung von Daten zeigen und/oder speichern und/oder verarbeiten, und
das Wasser entlang der Messungslinie (50) über einen Abflussleitungsstutzen (12) zur Ableitung des Wassers aus der Vorrichtung ableiten.

13. Ein Verfahren nach Patentanspruch 12 **dadurch gekennzeichnet, dass** der Wasserdruck mit einer den Druck herabsetzenden Pumpe (4) zum Ausgleichen des Wasserdrucks auf eine gewünschte Höhe kontrolliert wird, bevor das Wasser in den Behälter (6) zur Entfernung von Bläschen geleitet wird.

14. Ein Verfahren nach Patentanspruch 12 oder 13 **dadurch gekennzeichnet, dass** Lagedatenmittel (18, 20) mit mindestens einem Messsensor (8, 10) und/oder mit den Mitteln (22, 24) zur Handhabung von Daten verbunden werden, um die Messresultate mit den Lagedaten in Verbindung zu bringen.

15. Ein Verfahren nach einem der Patentansprüche 12 bis 14 **dadurch gekennzeichnet, dass** Wasser derart in den Behälter (6) zur Entfernung von Bläschen geleitet wird, dass das Wasser in dem Behälter (6) zur Entfernung von Bläschen in eine rotierende Bewegung gezwungen wird, wobei Luft- und/oder Gasbläschen in die Mitte eines entstandenen Strudels getrieben werden, von wo aus sie entfernt werden.

16. Ein Verfahren nach einem der Patentansprüche 12 bis 15 **dadurch gekennzeichnet, dass** das zu messende Wasser in die Vorrichtung geleitet wird, indem mindestens ein Zuflussleitungsstutzen (2) das Wasser entlang in einer Wasserstraße, einem Wassergebiet oder Ähnlichem bewegt wird und/oder das Wasser mit einer Pumpe aus der Wasserstraße, dem Wassergebiet oder Ähnlichem aufgesogen wird.

## Revendications

1. Appareil destiné à mesurer de manière continue la qualité de l'eau dans une voie d'eau, une zone d'eau ou similaire à partir d'un bateau de mer qui se déplace, **caractérisé en ce que** l'appareil comprend : un accessoire de canalisation d'entrée de l'eau (2) installé à partir du vaisseau dans une voie d'eau, une zone d'eau ou similaire, destiné à amener l'eau à mesurer vers l'appareil au moyen d'une canalisation de mesure (50) pendant que le bateau se déplace ; au moins un capteur de mesure (8, 10) destiné à exécuter des mesures de l'eau amenée dans l'appareil ; des moyens de gestion de données (22, 24) destinés à afficher et/ou à stocker et/ou à traiter les données mesurées ; et un accessoire de canalisation de sortie de l'eau (14) destiné à évacuer hors de l'appareil l'eau qui est passée dans la canalisation de mesure (50) ; et une chambre d'élimination des bulles (6) installée dans la canalisation de mesure (50) avant les capteurs de mesure (8, 10) destinée à éliminer des bulles d'air et/ou de gaz contenues dans l'eau amenée dans la canalisation de mesure (50) pour une mesure continue de la qualité de l'eau dans la voie d'eau, la zone d'eau ou similaire.

2. Appareil selon la revendication 1, **caractérisé en ce qu'**il comprend en outre une pompe de dépressurisation (4) destinée à égaliser la pression de l'eau transportée dans la canalisation de mesure (50).

3. Appareil selon la revendication 1 ou la revendication 2, **caractérisé en ce qu'**il comprend en outre des moyens de données d'emplacement (18, 20) destinés à associer les résultats de mesure obtenus à partir de l'eau à mesurer aux données d'emplacement.

4. Appareil selon l'une quelconque des revendications 1 à 3, **caractérisé en ce que** la chambre d'élimination des bulles (6) comprend : un accessoire de canalisation d'entrée (28) destiné à approvisionner en eau la chambre d'élimination des bulles (6) ; un accessoire de canalisation de sortie (30) destiné à évacuer l'eau à partir de la chambre d'élimination des bulles (6) ; et un accessoire de canalisation d'élimination des bulles (32) destiné à éliminer les bulles de la chambre d'élimination des bulles (6).

5. Appareil selon l'une quelconque des revendications 1 à 4, **caractérisé en ce que** la chambre d'élimination des bulles (6) est conçue de telle sorte, et l'accessoire de canalisation d'entrée (28) de la chambre d'élimination des bulles (6) est placé de telle sorte, que l'eau qui circule dans la chambre (6) soit forcée à suivre un mouvement de rotation dans la chambre (6).

6. Appareil selon l'une quelconque des revendications 1 à 5, **caractérisé en ce que** la section transversale de la chambre d'élimination des bulles (6) dans le sens de la circulation de l'eau qui pénètre dans la chambre d'élimination des bulles (6) par l'intermédiaire de l'accessoire de canalisation d'entrée (28), est sensiblement circulaire.

7. Appareil selon la revendication 6, **caractérisé en ce que** la chambre d'élimination des bulles (6) est sensiblement cylindrique.

8. Appareil selon la revendication 6, **caractérisé en ce que** la chambre d'élimination des bulles (6) est sensiblement conique.

9. Appareil selon l'une quelconque des revendications 1 à 8, **caractérisé en ce que** l'accessoire de canalisation d'entrée (28) de la chambre d'élimination des bulles (6) est placé dans la partie inférieure d'une cloison de chemise (36) de la chambre d'élimination des bulles (6) au-dessus d'une cloison inférieure (34) de manière tangentielle par rapport à la cloison de chemise (36) de telle sorte que l'eau qui pénètre dans la chambre soit forcée à suivre un mouvement de rotation.

10. Appareil selon l'une quelconque des revendications 1 à 9, **caractérisé en ce que** l'accessoire de canalisation d'élimination des bulles (32) est placé dans la chambre d'élimination des bulles (6) de manière coaxiale avec l'axe du milieu d'un tourbillon créé dans la chambre d'élimination des bulles (6).

11. Appareil selon l'une quelconque des revendications 1 à 10, **caractérisé en ce que** l'accessoire de canalisation de sortie (30) est placé dans la cloison inférieure (30) de la chambre d'élimination des bulles (6).

12. Procédé destiné à mesurer de manière continue la qualité de l'eau dans une voie d'eau, une zone d'eau ou similaire à partir d'un bateau de mer qui se déplace, comprenant les étapes suivantes consistant à :
✔ prélever de manière continue de l'eau à mesurer à partir de la voie d'eau, de la zone d'eau ou similaire dans l'appareil par l'intermédiaire d'un accessoire de canalisation d'entrée de l'eau (2) lorsque le bateau se déplace ;
✔ amener l'eau prélevée dans l'appareil le long d'une canalisation de mesure (50) dans une chambre d'élimination des bulles (6) de façon à éliminer toutes les bulles d'air et/ou de gaz contenues dans l'eau avant d'exécuter des mesures de l'eau prélevée dans l'appareil ;
✔ amener l'eau, dont les bulles d'air et/ou de gaz ont été éliminées, le long de la canalisation de mesure (50) vers au moins un capteur de mesure (8, 10) de façon à exécuter de manière continue des mesures de l'eau amenée dans l'appareil ;
✔ afficher et/ou stocker et/ou traiter les données mesurées avec les capteurs de mesure (8, 10) au moyen de moyens de gestion des données (22, 24) ; et
✔ amener l'eau le long de la canalisation de mesure (50) par l'intermédiaire d'un accessoire de canalisation d'évacuation de l'eau (12) hors de l'appareil.

13. Procédé selon la revendication 12, **caractérisé par** une étape consistant à commander la pression de l'eau avec une pompe de dépressurisation (4) avant de l'amener dans la chambre d'élimination des bulles (6) de façon à égaliser la pression de l'eau à un niveau désiré.

14. Procédé selon la revendication 12 ou la revendication 13, **caractérisé par** une étape consistant à connecter des moyens de données d'emplacement (18, 20) à un capteur de mesure (8, 10) au moins et/ou aux moyens de gestion de données (22, 24) de façon à associer les résultats de mesure aux données d'emplacement.

15. Procédé selon l'une quelconque des revendications 12 à 14, **caractérisé par** une étape consistant à amener l'eau à la chambre d'élimination des bulles (6) de façon à forcer l'eau à suivre un mouvement de rotation dans la chambre (6), grâce à quoi toutes les bulles d'air et/ou de gaz sont entraînées au milieu d'un tourbillon créé, d'où elles sont éliminées.

16. Procédé selon l'une quelconque des revendications 12 à 15, **caractérisé par** une étape consistant à prélever l'eau à mesurer dans l'appareil en déplaçant au moins l'accessoire de canalisation d'entrée de l'eau (2) par rapport à l'eau dans la voie d'eau, la zone d'eau ou similaire et/ou en utilisant une pompe de façon à aspirer de l'eau à partir de la voie d'eau, de la zone d'eau ou similaire.
